# EUROPEAN PATENT SPECIFICATION

(11) **EP 2 717 776 B1**
(45) Date of publication and mention of the grant of the patent: **26.07.2017**
(21) Application number: 12727524.6
(22) Date of filing: 04.06.2012
(51) Int. Cl.: A61M 5/28, A61B 5/1455, A61M 5/32, A61B 5/157, A61B 5/15, A61B 5/1495, A61B 5/151, A61M 5/31, A61B 90/90

(54) **Integrated lancing device**
Vorrichtung mit integrierter Lanzette
Dispositif de prélèvement intégré

(30) Priority: 09.06.2011 US 201113156491
(43) Date of publication of application: 16.04.2014
(73) Proprietor: Sanofi-Aventis Deutschland GmbH, 65929 Frankfurt am Main (DE)
(72) Inventor: CASTLE, Mark, Pleasanton, CA 94588 (US); KUGIZAKI, Rodney, Tucson, AZ 85755 (US)
(74) Representative: McDougall, James
(86) International application number: PCT/US2012/040740
(87) International publication number: WO 2012/170348

(56) References cited:
- EP-A1- 1 486 766
- WO-A1-2010/109461
- WO-A1-2011/041449
- US-A- 6 093 156
- US-A1- 2006 094 986
- US-A1- 2009 270 765
- US-A1- 2011 077 478

## Description

### BACKGROUND OF THE INVENTION

Lancing devices are known in the medical health-care products industry for piercing the skin to produce blood for analysis. Biochemical analysis of blood samples is a diagnostic tool for determining clinical information. Many point-of-care tests are performed using whole blood, the most common being monitoring diabetic blood glucose level. Other uses for this method include the analysis of oxygen and coagulation based on Prothrombin time measurement. Typically, a drop of blood for this type of analysis is obtained by making a small incision in the fingertip, creating a small wound, which generates a small blood droplet on the surface of the skin.

Early methods of lancing included piercing or slicing the skin with a needle or razor. Current methods utilize lancing devices that contain a multitude of spring, cam and mass actuators to drive the lancet. These include cantilever springs, diaphragms, coil springs, as well as gravity plumbs used to drive the lancet. Typically, the device is pre-cocked or the user cocks the device. The device is held against the skin and the user, or pressure from the users skin, mechanically triggers the ballistic launch of the lancet. The forward movement and depth of skin penetration of the lancet is determined by a mechanical stop and/or dampening, as well as a spring or cam to retract the lancet. Such devices have the possibility of multiple strikes due to recoil, in addition to vibratory stimulation of the skin as the driver impacts the end of the launcher stop, and only allow for rough control for skin thickness variation. Different skin thickness may yield different results in terms of pain perception, blood yield and success rate of obtaining blood between different users of the lancing device.

Success rate generally encompasses the probability of producing a blood sample with one lancing action, which is sufficient in volume to perform the desired analytical test. The blood may appear spontaneously at the surface of the skin, or may be "milked" from the wound. Milking generally involves pressing the side of the digit, or in proximity of the wound to express the blood to the surface. In traditional methods, the blood droplet produced by the lancing action must reach the surface of the skin to be viable for testing.

When using existing methods, blood often flows from the cut blood vessels but is then trapped below the surface of the skin, forming a hematoma. In other instances, a wound is created, but no blood flows from the wound. In either case, the lancing process cannot be combined with the sample acquisition and testing step. Spontaneous blood droplet generation with current mechanical launching system varies between launcher types but on average it is about 50% of lancet strikes, which would be spontaneous. Otherwise milking is required to yield blood. Mechanical launchers are unlikely to provide the means for integrated sample acquisition and testing if one out of every two strikes does not yield a spontaneous blood sample.

Many diabetic patients (insulin dependent) are required to self-test for blood glucose levels five to six times daily. The large number of steps required in traditional methods of glucose testing ranging from lancing, to milking of blood, applying blood to the test strip, and getting the measurements from the test strip discourages many diabetic patients from testing their blood glucose levels as often as recommended. Tight control of plasma glucose through frequent testing is therefore mandatory for disease management. The pain associated with each lancing event further discourages patients from testing. Additionally, the wound channel left on the patient by known systems may also be of a size that discourages those who are active with their hands or who are worried about healing of those wound channels from testing their glucose levels.

Another problem frequently encountered by patients who must use lancing equipment to obtain and analyze blood samples is the amount of manual dexterity and hand-eye coordination required to properly operate the lancing and sample testing equipment due to retinopathies and neuropathies particularly, severe in elderly diabetic patients. For those patients, operating existing lancet and sample testing equipment can be a challenge. Once a blood droplet is created, that droplet must then be guided into a receiving channel of a small test strip or the like. If the sample placement on the strip is unsuccessful, repetition of the entire procedure including re-lancing the skin to obtain a new blood droplet is necessary. Examples of sampling devices can be seen in US 2011/077478, US 2009/270765; WO 2010/109461; and US 6,093,156. An example of a test strip for use on a fluid sample is described in BP 1486766.

### SUMMARY OF THE INVENTION

The invention is defined by the claims.

### BRIEF DESCRIPTION OF THE DRAWINGS

FIGS. 116(a) and 116(b) illustrate top and side views of examples of a lancet driver with a multi-switch user interface of the present invention.
FIG. 117 illustrates an example of a lancet driver with an LED.
FIG. 118 illustrates an example of a lancet driver of the present invention with a semi-transparent lancet window.
FIGS. 119(a)-119(c) illustrate an embodiment of a lancing device that provides integrated lancing and body fluid measurement.

### DESCRIPTION OF THE SPECIFIC EMBODIMENTS

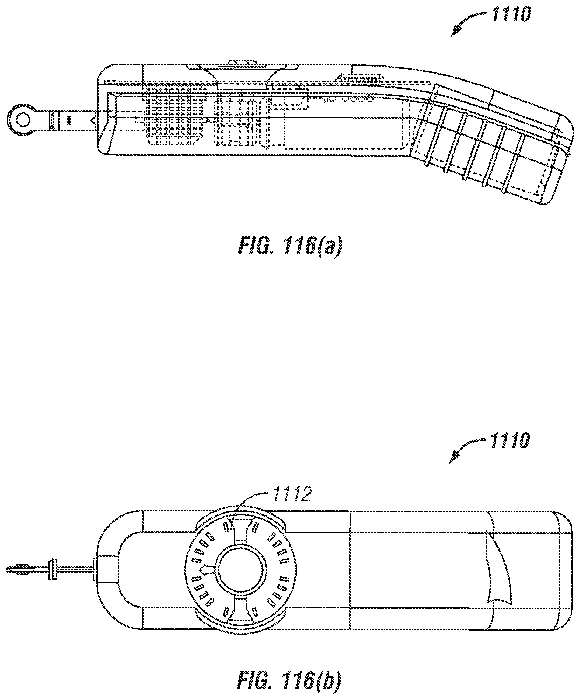

Referring to FIGS. 116(a) and 116(b), one example of a lancing device 1110 is provided that includes, (i) a manual switch for a user interface input, (ii) an LED or light source, (iii) a user interface indicator, (iv) a transparent lancet detect window and (v) angled cylindrical housings.

The multi-position mechanical switch 1112 is illustrated in FIGS. 116(a) and 116(b). The multi-position mechanical switch 1112 can have fixed or mechanically indexed positions via low cost and high reliability circuit board contact pads. These provide a digital switch connection or combined analog electronic level and used as a user interface input control. The use interface input control can provide for depth setting range and comfort profile which, as a non-limiting example, can be about 3 to 100 discrete steps, provide device on and off, device standby, and the like. It also can enable lancing device 1110 to be put in a sleep or standby mode as well as disable launching of the lancet unintentionally with the fire button.

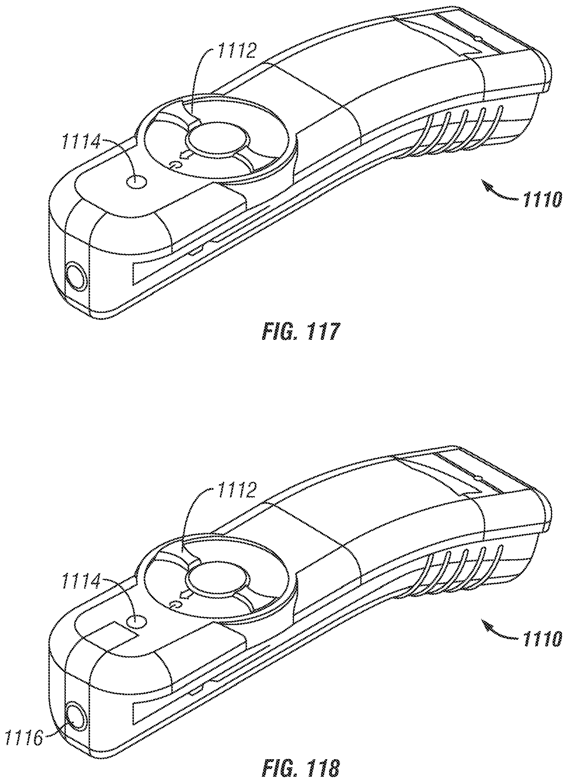

An LED, or other suitable light source 1114, shown in FIG. 117, can be used to indicate a variety of different user interface outputs including but not limited to, low battery, charging, lancet present, device error condition(s), ready to launch, an indication that the battery requires replacement and the like. A clear, semi-transparent and/or molded housing feature permits light or partial light transmittance of symbols, including but not limited to, a battery annucator, lancet present symbol, audio on/off, depth setting, data management mode and the like.

A clear or semitransparent housing window 1116, illustrated in FIG. 118, allows the user to visibly confirm that a lancet is loaded, unloaded, moving during launch, and enables a secondary function of enabling removal of physical contaminants such as blood, dust or other objects potentially detrimental to the bearing actuator and cleaning by removing the housing window.

In one example, the lancing device 1110 has angled housings. This provides for a smaller device volume via two dissimilar cylindrical shapes. One shape is for a larger radial battery, and the other smaller diameter is for all additional hardware such as the Actuator Assembly, high voltage capacitor, PCB and all electrical components, transformer, encoder, lancets, microcontroller.

In one example, the lancing device 1110 has the following design requirements/specifications: (i) Mass: low mass, which as a non-limiting example can be a total moving mass < 0.40 g (ii) Friction: very low, consistent friction (affects contact point) (iii) user interface: simple, intuitive, requiring very low dexterity, visual acuity and tactile feel. Some design constraints include but are not limited to, (i) user handled, over-molded lancet needle, (ii) a sterility barrier provided by overmold, removed by user, and the like.

In various examples, design elements of the lancing device 1110 can include but are not limited to, (i) lancet-chuck coupling: robust, accuracy of needle placement X-Y-Z, tactile feedback for insertion and removal; (ii) lancet present/absent detection: low cost solution to detecting presence/absence; (ii) interface to lancing drive: close mechanical coupling for drive (slug), (iii) bearings/guidance: per tolerance analysis, varies for application, (iv) latching: ability to latch for storage, removal of lancet, and the like.

In various examples, design elements of the lancing device 1110 include but are not limited to, (i) lancet-chuck coupling: method for interfacing removable over-molded lancet to chuck/drive; (ii) toroidal spring retention (e.g., balseal); (iii) bearings: guide feature(s) for accurately defining lancing trajectory; (iv) chuck-chassis form bearings; (v) bearings contained in a disposable cartridge; (vi) latching: holding the lancet from exiting the lancing device 1110 when not in use and for removal of the lancet; (vii) a magnetic actuated latch; (viii) a button press for lancet access, latching; (ix) lancet present detection: transparent detection of lancet present or absent from the lancing device 1110; (x) moving a plunger for an encoder relative to a chuck (lancet insertion positions encoder to home position relative to chuck); (xi) lancing device 1110 being capable of taking action based on presence or absence of elements; (xii) visual (user observes) detection of the lancet; (xiii) load-unload of the lancet; (xiv) lancet detection through an aperture; (xv) user inserted lancet cartridge, and the like.

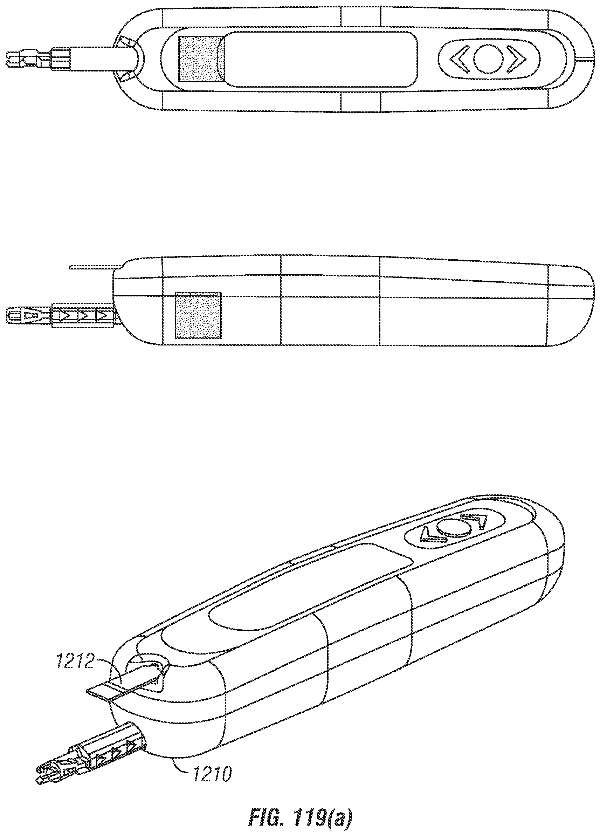

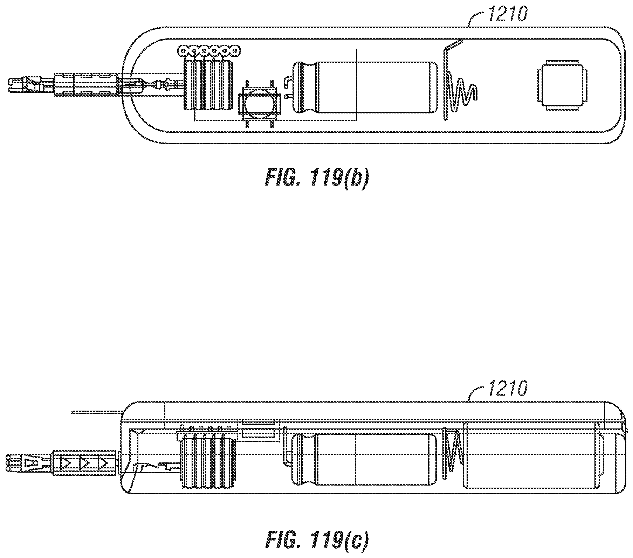

In another embodiment of the present invention, illustrated in Figures 119(a)-119(c), a lancing device 1210 is provided with an aperture 1212 that receives a test strip. Suitable test strips are disclosed in U.S. Patent No. 6,555,061. In one embodiment, the lancing device 1210 is essentially more of a lancing device instead of a body fluid meter with a lancet in it. The lancing device 1210 can include some or all of the electronics, and analyte sensors disclosed in U.S. Publication No. 2005120365.

The lancing device 1210 can be sized to be able to be placed easily in a pocket. The lancing device 1210 can have a vertical mount geometry with a test strip aperture on top of an exit for a lancet, with the two positioned in-line. In one embodiment, the lancing device 1210 has a geometry and layout to provide that a lanced finger only moves about 25mm from the lancing element to the test strip.

The lancing device 1210 is painless, provides a discrete measurement, provides finger health, ease of testing, and integrates lancing with body fluid measure in a single device. There is essentially no sound.

The lancing device 1210 provides a number of advantages including but not limited to, (i) a one touch test from lancing of a tissue site, such as a finger or the forearm, following by movement of the finger that now has a drop of blood a short distance to a test strip positioned about the lancet, (ii) a small sample size of 1 microliter or less, (iii) date and time information displaying, (iv) no cleaning, (v) it is ergonomic, (vii) inexpensive, (viii) very robust, and the like. In one embodiment, the lancing device 1210 has 2-way scrolling buttons for simple navigation.

The lancing device 1210 can have a large test memory. As a non-limiting example, the test memory can be a 500 test memory to review prior results. The lancing device 1210 can have a relative large display with a back light, and testing can be completed in less than 10 seconds, more preferable 5 seconds or less.

In various embodiments, the internal components of the lancing device 1210 can be arrange in a, (i) single wide with substantially all components in a linear path, (ii) double wide, and (iii) triple wide arrangements. As a non-limiting example, all of the electronic elements can be arranged in a single wide configuration. As a non-limiting example, a housing of the lancing device can have the following dimensions, width of 27 mm, height of 24 mm, and a length of 127 mm. More than one battery can be provided. Two batteries can be included and be positioned in a portion of the lancing device 1210 than can have a double wide geometry coupled to a single wide portion.

The positioning of the LCD screen for the human interface may be varied so as to provide the best location for ergonomic use. The human interface may be a voice system that uses words to describe status or alarms related to device usage.

## Claims

1. A lancing device, **characterised by** comprising:
a housing with a length greater than a width;
electronics positioned in an interior of the housing;
a test strip aperture (1212) in the housing adapted to receive a body fluid test strip;
a lancet aperture positioned in line with the test strip aperture (1212); and
a test memory that in operation provides for review of prior results.

2. The lancing device of claim 1, wherein a lancet (72, 1002) is positioned in the housing and a sharpened end of the lancet exits from the lancet aperture.

3. The lancing device of claim 1 or claim 2, wherein the lancing device (1210) integrates lancing and testing of a body fluid in a single device.

4. The lancing device of claim 1 or claim 2, wherein the lancing device (1210) wherein the lancet (72, 1002) has an impact velocity of greater than 2 m/s when used on a tissue site (234).

5. The lancing device of any preceding claim, further comprising:
an electronic driver (68, 1000) positioned in the housing, the electronic driver (68, 1000) configured to be coupled to the lancet (72, 1002);
a processor (60, 1020) coupled to the electronic driver (68, 1000), the processor (60, 1020) storing user profiles (62) in a memory that are customizable in response to user input information (64).

6. The lancing device of claim 5, wherein the processor (60, 1020) is coupled to or illustrates a database (80).

7. The lancing device of claim 5, wherein the profile traits are selected from at least one of, degree of painlessness, success rate, and blood volume.

8. The lancing device of claim 6 or claim 7, further comprising:
an internal clock that allows storage in the database that includes information selected from at least one of, time of day for a lancing event and s time between lancing events.

9. The lancing device of any one of claims 6 to 8, wherein the database (80) stores information and statistics for each user and each profile (62) that a particular user uses.

10. The lancing device of claim 5, or any one of claims 6 to 9 when read dependent on claim 5, wherein the processor (60, 1020) is configured to calculate an appropriate lancet diameter and geometry necessary to realize a blood volume required for a user.

11. The lancing device of claim 5, or any one of claims 6 to 9 when read dependent on claim 5, wherein for each of a class of lancet, diameter and lancet geometry are stored in the processor (60, 1020) to correspond with upper and lower limits of attainable blood volume.

12. The lancing device of any preceding claim, further comprising:
a housing member (1114) configured to permits light or partial light transmittance of symbols.

13. The lancing device of claim 12, wherein the symbols are selected from at least one of, battery status, lancet present symbol, audio on/off, depth setting and data management mode.

## Patentansprüche

1. Lanzettenvorrichtung, **dadurch gekennzeichnet, dass** sie umfasst:
ein Gehäuse mit einer Länge, die größer ist als eine Breite;
eine Elektronik, die in einem Inneren des Gehäuses positioniert ist;
eine Teststreifenöffnung (1212) in dem Gehäuse, die so ausgelegt ist, dass sie einen Körperflüssigkeits-Teststreifen aufnimmt;
eine Lanzettenöffnung, die in Übereinstimmung mit der Teststreifenöffnung (1212) positioniert ist; und
einen Testspeicher, der bei Betrieb eine Überprüfung von vorhergehenden Ergebnissen bietet.

2. Lanzettenvorrichtung nach Anspruch 1, wobei eine Lanzette (72, 1002) in dem Gehäuse positioniert ist und ein spitzes Ende der Lanzette aus der Lanzettenöffnung austritt.

3. Lanzettenvorrichtung nach Anspruch 1 oder Anspruch 2, wobei in der Lanzettenvorrichtung (1210) das Lanzettieren und das Testen einer Körperflüssigkeit in einer einzelnen Vorrichtung integriert sind.

4. Lanzettenvorrichtung nach Anspruch 1 oder Anspruch 2, wobei die Lanzettenvorrichtung (1210), wobei die Lanzette (72, 1002) eine Auftreffgeschwindigkeit von mehr als 2 m/s aufweist, wenn sie an einer Gewebestelle (234) verwendet wird.

5. Lanzettenvorrichtung nach einem der vorhergehenden Ansprüche, die ferner umfasst:
einen Elektroniktreiber (68, 1000), der in dem Gehäuse positioniert ist, wobei der Elektroniktreiber (68, 1000) so ausgeführt ist, dass er mit der Lanzette (72, 1002) gekoppelt ist;
einen Prozessor (60, 1020), der mit dem Elektroniktreiber (68, 1000) gekoppelt ist, wobei der Prozessor (60, 1020) Nutzerprofile (62) in einem Speicher speichert, die in Reaktion auf Nutzereingabeinformationen (64) kundenspezifisch ausgebildet werden können.

6. Lanzettenvorrichtung nach Anspruch 5, wobei der Prozessor (60, 1020) mit einer Datenbank (80) gekoppelt ist oder diese darstellt.

7. Lanzettenvorrichtung nach Anspruch 5, wobei die Profilmerkmale ausgewählt sind aus mindestens einem von Grad an Schmerzlosigkeit, Erfolgsrate und Blutvolumen.

8. Lanzettenvorrichtung nach Anspruch 6 oder Anspruch 7, die ferner umfasst:
eine interne Uhr, die eine Speicherung in der Datenbank ermöglicht, die Informationen aufweist, welche ausgewählt sind aus mindestens einem von Tageszeit für einen Lanzettiervorgang und s-Zeit zwischen Lanzettiervorgängen.

9. Lanzettenvorrichtung nach einem der Ansprüche 6 bis 8, wobei die Datenbank (80) Informationen und Statistiken für jeden Nutzer und jedes Profil (62), das ein bestimmter Nutzer verwendet, speichert.

10. Lanzettenvorrichtung nach Anspruch 5 oder einem der Ansprüche 6 bis 9 mit Rückbezug auf Anspruch 5, wobei der Prozessor (60, 1020) so ausgeführt ist, dass er einen angemessenen Lanzettendurchmesser und eine angemessene Lanzettengeometrie berechnet, die zum Realisieren eines Blutvolumens notwendig sind, das für einen Nutzer erforderlich ist.

11. Lanzettenvorrichtung nach Anspruch 5 oder einem der Ansprüche 6 bis 9 mit Rückbezug auf Anspruch 5, wobei für jede Klasse von Lanzetten Durchmesser und Lanzettengeometrie in dem Prozessor (60, 1020) gespeichert sind, um mit dem oberen und dem unteren Grenzwert eines erzielbaren Blutvolumens übereinzustimmen.

12. Lanzettenvorrichtung nach einem der vorhergehenden Ansprüche, die ferner umfasst:
ein Gehäuseelement (1114), das so ausgeführt ist, dass es das Durchlassen von Licht oder Teillicht von Symbolen ermöglicht.

13. Lanzettenvorrichtung nach Anspruch 12, wobei die Symbole ausgewählt sind aus mindestens einem von Batteriestand, Lanzettevorhanden-Symbol, Audio ein/aus, Tiefeneinstellung und Datenmanagementmodus.

## Revendications

1. Dispositif de prélèvement, **caractérisé en ce qu'**il comprend :
un boîtier ayant une longueur supérieure à une largeur ;
des dispositifs électroniques positionnés à l'intérieur du boîtier ;
une ouverture de bande de test (1212) pratiquée dans le boîtier, adaptée pour recevoir une bande de test de fluide corporel ;
une ouverture de lancette positionnée en ligne avec l'ouverture de bande de test (1212) ; et
une mémoire de test qui, en opération, permet d'examiner les résultats antérieurs.

2. Dispositif de prélèvement selon la revendication 1, dans lequel une lancette (72, 1002) est positionnée dans le boîtier, et une extrémité aiguisée de la lancette sort de l'ouverture de la lancette.

3. Dispositif de prélèvement selon la revendication 1 ou la revendication 2, dans lequel le dispositif de prélèvement (1210) intègre les opérations de prélèvement et le test d'un fluide corporel dans un seul dispositif.

4. Dispositif de prélèvement selon la revendication 1 ou la revendication 2, dans lequel le dispositif de prélèvement (1210) dans lequel la lancette (72, 1002) a une vitesse d'impact supérieure à 2 m/s lorsqu'elle est utilisée sur un site de tissu (234).

5. Dispositif de prélèvement selon l'une quelconque des revendications précédentes, comprenant en outre :
un organe de commande électronique (68, 1000) positionné dans le boîtier, l'organe de commande électronique (68, 1000) étant configuré pour être couplé à la lancette (72, 1002) ;
un processeur (60, 1020) couplé à l'organe de commande électronique (68, 1000), le processeur (60, 1020) stockant des profils d'utilisateur (62) dans une mémoire, lesquels sont personnalisables en réponse à des informations entrées par un utilisateur (64).

6. Dispositif de prélèvement selon la revendication 5, dans lequel le processeur (60, 1020) est couplé à, ou illustre, une base de données (80).

7. Dispositif de prélèvement selon la revendication 5, dans lequel les traits de profil sont sélectionnés parmi au moins un degré d'absence de douleur, un taux de réussite et un volume sanguin.

8. Dispositif de prélèvement selon la revendication 6 ou la revendication 7, comprenant en outre :
une horloge interne qui permet un stockage dans la base de données qui comprend des informations sélectionnées parmi au moins l'une des suivantes : l'heure du jour d'un événement de prélèvement et le temps entre les événements de prélèvement.

9. Dispositif de prélèvement selon l'une quelconque des revendications 6 à 8, dans lequel la base de données (80) stocke des informations et des statistiques pour chaque utilisateur et chaque profil (62) qu'un utilisateur particulier utilise.

10. Dispositif de prélèvement selon la revendication 5 ou l'une quelconque des revendications 6 à 9 lorsqu'elles sont lues en lien avec la revendication 5, où le processeur (60, 1020) est configuré pour calculer un diamètre et une géométrie de lancette appropriés nécessaires pour obtenir un volume de sang requis pour un utilisateur.

11. Dispositif de prélèvement selon la revendication 5, ou l'une quelconque des revendications 6 à 9 lorsqu'elles sont lues en lien avec la revendication 5, où, pour chacune d'une classe de lancette, le diamètre et la géométrie de la lancette sont stockés dans le processeur (60, 1020) pour correspondre aux limites supérieures et inférieures du volume sanguin atteignable.

12. Dispositif de prélèvement selon l'une quelconque des revendications précédentes, comprenant en outre :
un élément de boîtier (1114) configuré pour permettre une transmission lumineuse ou une transmission lumineuse partielle de symboles.

13. Dispositif de prélèvement selon la revendication 12, dans lequel les symboles sont sélectionnés parmi au moins un des suivants : l'état de la batterie, un symbole de présence de lancette, l'activation/la désactivation du son, le réglage de la profondeur et un mode de gestion des données.
